# EUROPEAN PATENT SPECIFICATION

(11) **EP 4 147 785 B1**
(45) Date of publication and mention of the grant of the patent: **02.04.2025**
(21) Application number: 21195980.4
(22) Date of filing: 10.09.2021
(51) Int. Cl.: B04B 1/08, B04B 5/10, B04B 11/02

(54) **METHOD OF CONCENTRATING A PLANT-BASED PROTEIN SUSPENSION**
VERFAHREN ZUR KONZENTRATION EINER SUSPENSION AUF PFLANZLICHER BASIS
PROCÉDÉ DE CONCENTRATION D'UNE SUSPENSION DE PROTÉINE À BASE DE PLANTES

(43) Date of publication of application: 15.03.2023
(73) Proprietor: Alfa Laval Corporate AB, 221 00 Lund (SE)
(72) Inventor: LINNET, Lars, DK-3000 HELSINGØR (DK); MASIK, Joosep, DK-2800 Kgs. Lyngby (DK)
(74) Representative: Alfa Laval Attorneys

(56) References cited:
- EP-A1- 2 868 210
- CN-A- 112 841 392
- DE-C- 839 594

## Description

### FIELD OF THE INVENTION

The present disclosure relates to a method of concentrating a plant-based protein suspension as defined in the appended claims.

### BACKGROUND

The global population is continuously growing, thereby increasing the demand for proteins as food. Studies show that by 2050 the global population reach will nearly 10 billion - an increase of 2.3 billion people. As a result, the demand for protein will increase by 50%, corresponding to more than 256 million tons per annum. At the same time, the arable land and other resources needed to produce traditional meat proteins is limited. These are the two main challenges that require new solutions, i.e., to provide protein for growing population with reduced resources.

Plant-based proteins are considered as one solution that may address these challenges. US2284700A discloses a method of preparing a composite soya bean product which comprises leaching soya bean meal containing water soluble proteins, water soluble carbohydrates, alkali soluble globulin proteins, hemicellulose, cellulose and insoluble prolamine proteins with a weak acid at a pH corresponding to the isoelectric point of the alkali soluble globulin proteins in the meal, dissolving the alkali soluble proteins from the meal, separating the composite of insoluble residue and precipitated protein from the solution by decanting, and drying the composite thus formed.

However, there are challenges in the existing processes, in which protein precipitate suspensions are recovered. To recover the precipitated proteins, decanter centrifuges is normally used. However, as some plant-based protein precipitate suspensions are very light, there is a risk for overflow of fine suspensions to a centrate, and therefore flowrates need to be reduced. Therefore, to keep up with the desired capacity, the number of decanter centrifuges is often increased, which is not desirable.

There is thus a desire to provide improved high-capacity ways to provide concentrated protein suspensions.

### SUMMARY

In view of the capacity problem mentioned above, it has been noted to be challenging to increase capacity without increasing the number of decanters. Further, it has been noted different plant-based protein precipitates have different characteristics: some of the precipitates are soft and bulky having a great volume in proportion to weight and such precipitates are more difficult to separate than compact precipitates. This problem has been realized especially when concentrating plant-based protein other than soy. When using other plant-based raw materials, such as pea or mung bean as protein sources, the precipitates may become soft and bulky. The decanting and recovery of such precipitated proteins can be very demanding, since they are not easily compacted into a sediment that can be scrolled out of the decanter. When a decanter centrifuge has difficulties in scrolling out the solids, the bowl gradually fills up and as the clarifying volume decreases, the overflow of fine suspended particles into the centrate increases. Therefore, the flowrate to the decanter needs to be decreased and the capacity of the decanter is decreased. This in turn results in many parallel decanters at this process step.

High-speed centrifugal separators with a separation space comprising a disc stack have a high capacity and can provide separation of a material to a solid phase, liquid heavy phase and liquid light phase. Plant-based protein processing systems may employ discharging centrifuges of clarifier type for carried-over fines recovery or for a secondary recovery of whey proteins (albumins not precipitating at the isoelectric point) after thermal coagulation. However, continuously discharging 3-phase high speed separators have not been used for separating precipitated suspended plant-based proteins. Such suspended proteins are soft and bulky in the suspension, and it is difficult to form a clear interface between the liquid and the suspended particles in a 3-phase high speed centrifugal separators. Additionally, in connection with soybean and groundnut or other oilseeds it has been described by GB813434 that precipitated suspension thereof tend to block the nozzles of a centrifuge, whereby there is a need to add salt before Centrifugal separation is also known from CN-A-112,841,392; DE-A-839,594 and EP-A-2,868,210.

Despite the teachings away from the present invention, the inventors have noted that it is possible to use high-speed separation for recovering a concentrated plant-based protein suspension having bulky and soft precipitates. By concentrated suspension is meant a suspension from which clear liquid has been at least partly removed. It has been noted that such suspensions and precipitates have a shear thinning characteristics, meaning that the viscosity decreases with increasing shear rate. It was then surprisingly found that use can be made of a 3-phase high speed centrifugal separator, having means to influence, i.e. actively or passively regulate, the flow through a heavy phase outlet such that a concentrated protein suspension comprising the precipitated bulky protein particles is conveyed through the heavy phase outlet, and that a liquid light phase, which corresponds to clarified liquid of the suspension, is conveyed through the liquid light phase outlet and as defined in the appended claims. By influencing the flow is meant that flow characteristics, such as counter pressure or movement, e.g., laminar-turbulent, characteristics are influenced by actively or passively regulating the flow characteristics. For example, valves may be incorporated to actively regulate the counter-pressure. Alternatively, or additionally, different types of passive vortex generating means can be used in fluid channels of the separator, e.g., at the heavy phase outlet of a separator.

Thus, the present invention relates to a method of recovering a concentrated plant-based protein suspension, which comprises providing a plant-based protein suspension comprising suspended particles, by dissolving a plant-based protein and by precipitating the dissolved protein to provide the plant-based protein suspension, and providing a high-speed centrifugal separator which comprises a frame, a drive member and a centrifuge bowl. The drive member is configured to rotate the centrifuge bowl in relation to the frame around an axis of rotation. The centrifuge bowl encloses a separation space comprising a stack of separation discs and comprises an inlet for receiving the plant-based protein suspension, a liquid light phase outlet for a separated liquid light phase and a heavy phase outlet for a separated heavy phase. The heavy phase outlet and/or light phase outlet is arranged in fluid connection with a flow influencing means. The method further comprises feeding the plant-based protein suspension to the inlet of the high-speed centrifugal separator, separating the plant-based protein suspension into a liquid light phase and a heavy phase, which comprises the concentrated protein suspension. The method comprises removing the concentrated plant-based protein suspension as the heavy phase through the heavy phase outlet by influencing the discharge flow by means of the flow influencing means.

The step of influencing the flow through the liquid heavy phase outlet may comprise adjusting counter pressure of the liquid heavy phase outlet with respect to the liquid light phase outlet, or vice versa, and wherein the liquid flow influencing means comprises a valve. The valve can be connected to the and/or light phase outlet.

An example of a suitable separator, in which the adjusting of the counter pressure can be done, is a separator arrangement in which both the light and heavy phases are continuously pumped out through a respective outlet with the help of a built-in paring disk from the separator. Such separator is especially suitable for suspensions comprising bulky protein precipitates. This mode of transport ensures that even the fine suspended particles can be hydrodynamically carried out from the machine and the g-force that is developed ensures that protein particles will accumulate at the periphery and from there will be directed to the top disk and eventually to the heavy phase outlet.

The flow influencing means may be a flow regulating means comprising a valve connected to the heavy and/or light phase outlet. The heavy phase outlet may comprise means for adjusting counter pressure of the heavy phase at the heavy phase outlet with respect to the liquid light phase outlet, or vice versa. Thus, the counter pressure on both the light and the heavy phase outlet can be adjusted. This working principle is fundamentally different from both a clarifier type of a separator with a regular discharge of solids from the periphery of a separator bowl, but also from a horizontal decanter centrifuge.

Alternatively, the heavy phase outlet may comprise a passive flow regulator, such as a vortex generator, tesla valve, and/or different types of nozzles that can influence or passively regulate the flow geometry and/or viscosity. An example of a suitable type of passive regulator is disclosed in US4311270, which shows a vortex fluidic device in the outlet of the separator, which is a passive regulator that influences the flow through the heavy phase outlet.

According to a variant, the method may further comprise measuring at least one parameter of the removed heavy phase and/or liquid light phase, wherein said parameter is related to the concentration of the heavy phase in the light phase, or vice versa; and adjusting the counter pressure of the heavy phase outlet with respect to the liquid light phase outlet, or vice versa, based on the parameter related to the concentration. In this way the concentration of the heavy phase in the liquid light phase, or vice versa, discharged from the separator can be controlled. The concentration of suspended protein particles present in the liquid light phase may be measured. It is desirable to keep this concentration as low as possible. The measurement may be performed manually off-line or in-line by means of an automatic sensor. In this way, the separation may be regulated so as to minimize the solids content in the light phase, whereby product losses can be minimized. The concentrated heavy phase may contain a larger content of liquid light phase to minimize the risk for clogging of the separator.

The method may comprise concentrating the plant-based protein suspension in at least two sequential centrifugal separation steps, wherein one of the two centrifugal separation steps is performed in a decanter centrifuge. By using a decanter centrifuge, the solids content in the separated heavy phase may be increased.

The first step of the centrifugal separation may be performed in the high-speed centrifugal separator and the second step may be performed in the decanter centrifuge. The use of the high-speed centrifugal separator may decrease product losses. Additionally, it may help to increase the capacity of the decanter. In this way, larger solids volumes can be handled in the first step. Alternatively, the first step of the centrifugal separation may be performed in the decanter centrifuge and a second step may be performed in the high-speed centrifugal separator. Thus, the high-speed separator will need to discharge less and may run even with a higher capacity.

The plant-based protein suspension may be provided by dissolving a plant-based protein and by precipitating the dissolved protein to provide the plant-based protein suspension. The plant-based protein may be dissolved by adding an alkali to a ground plant-based flour. The plant-based protein may be precipitated by adding an acid or an organic solvent, such as ethanol, to the clarified solution of dissolved plant-based protein.

According to a variant, the dissolved protein may be separated by means of mechanical separation from insoluble impurities and/or oil before precipitating the dissolved protein. In this way, the solids load in the centrifugal separating steps may be reduced.

According to a variant, no salt is added to the plant-based protein suspension after precipitation and before separation into a liquid light phase and a liquid heavy phase. The high-speed centrifugal separator in the step of separating may be mechanically hermetically sealed. Thereby oxygen take-up may be reduced, and a hygienic process provided.

The raw material for the suspension comprising plant-based proteins may comprise low-fat or high-fat plant-based raw materials, such as legumes, oilseeds and cereals. The raw material may comprise low-fat raw materials comprising legumes, such as yellow peas, fava beans, mung beans, lentils, chickpeas, or combinations thereof. Alternatively, the raw material may comprise high-fat raw materials comprising de-fatted oilseeds of soybean and lupin, sunflower, rapeseed, cottonseed, linseed or combinations thereof. The raw material is preferably other than soybean-based material. Such raw materials result in bulky/light precipitates in the protein suspensions, and the present method is especially suitable for concentrating such suspensions.

The method may comprise de-oiling the plant-based raw material before protein extraction. De-oiling is especially advantageous when the raw-material comprises high-fat raw materials. The de-oiling or de-fatting may be performed before the fractionation, i.e. the precipitation of the protein.

Further features, aspects and advantages with the present invention are described more in detail with reference to the attached figures.

### BRIEF DESCRIPTION OF DRAWINGS

Fig. 1 shows schematically a high-speed centrifugal separator suitable for the method of the present disclosure.
Fig. 2 shows schematically an example of a high-speed centrifugal separator.
Fig. 3 illustrates an example mode of the method.
Fig. 4 illustrates another example mode of the method.
Fig. 5 shows measured particle size distribution for the plant-based protein suspension to be concentrated.
Fig. 6 shows measured shear viscosity of both slurry (plant-based protein suspension) and solids.

### DETAILED DESCRIPTION

Liquid light and heavy phases and solids can be separated from each other by using centrifugal separators, which include decanters and high-speed disc stack separators, also referred to as centrifuges and/or high-speed separators. In the present method and system, both decanters and high-speed separators can be used in different stages of the process to achieve the required separation result. Centrifugal separation is advantageous if compared to e.g. filtration by pressing because the separation process is faster, it allows for more efficient separation of solids and liquids as it uses centrifugal force rather than pressing the product through openings in the filter, and it can be cleaned by CIP (Clean In Place) whereas the filters can normally not.

A decanter is a separator using centrifugal forces and can separate solids from one or two liquid phases. A decanter may be a so-called two-phase (2-phase) or three-phase (3-phase) decanter. The separation is performed by utilizing centrifugal forces that can be beyond 3000 times greater than gravity. When the material to be separated is subjected to such forces, the denser solid particles are pressed outwards against a rotating bowl wall, while the less dense liquid phase forms a concentric inner layer along a longitudinally extending central rotating screw conveyor inside the rotating bowl. Different dam or weir plates are used at the outlets to vary the depth of the liquid, the pond, as required. A sediment formed by the solid particles is continuously removed by the screw conveyor inside the decanter bowl, and the screw conveyor is arranged to rotate at a different speed than the bowl. As a result, the solids are gradually "ploughed" out of the pond and up a conical "beach". The centrifugal forces compact the solids and expels the surplus liquid. The dried solids are then discharged from the bowl. The clarified liquid phase or phases overflow the dam plates situated at the outlet on the opposite end of the bowl. The separated phases are then directed into the correct flow path to reduce or prevent a risk of cross-contamination. The speed of the screw conveyor may be automatically adjusted using a variable frequency drive (VFD) in order to adjust to a variation in the solids load. Decanter centrifuges can be used to remove large particles from slurries or liquids with a high concentration of solids and it is also possible to separate two liquid phases of varying densities. Decanters may have a separation range suitable for liquids containing more than e.g., 5-10% by weight solids. The particle size may be equal to or greater than 10 microns.

Disk stack separators, also referred to as high-speed separators, use centrifugal force to separate slurries that can have a lower concentration of solids than slurries separated by decanters, but can handle slurries with a high concentration of solids. High-speed separators can also handle slurries having relatively small particle sizes. High-speed separators are suitable for separating two liquid phases as well as a solids phase, and they can be configured as three-phase separators or two-phase separators. Different types of high-speed separators may be used for liquids with different solid contents. For example, continuously discharging nozzle centrifuge can handle higher solids loads than discharge-type of centrifugal separator, which discharges solids intermittently. The particle sizes can be between 0.1 micron (µm) and 150 microns. High-speed centrifugal separators comprising a disc stack are suitable for separating two liquid phases as well as a solids phase and the separation technology can be based on different densities of the liquid/solid phases. The high-speed or disc stack separators use mechanical forces to separate liquids and solids with different densities from each other. Rapid rotation of a separator bowl provides a centrifugal force, or gravitational force known as G-force, which can have an effect up to 10.000 times greater than the force of gravity. G-force is then used to separate liquids from other liquids and solids with accuracy and speed and the separation may be controlled. A disc stack within the bowl contributes to higher separation efficiency by increasing the separation area in the separator bowl. The solids that concentrate at the outer edge of the bowl are discharged - either continuously, intermittently or manually, depending on the volume of solids involved in the specific application. The high-speed separator may be hermetically sealed. For example, it may be hermetically sealed both at the inlets and the outlets, whereby a hygienic separation may be provided with reduced oxygen uptake.

There are basically three types of high-speed separators: a clarifier, purifier and concentrator. A clarifier is a centrifugal separator which may be used for solid/liquid separation, in which solids such as particles, sediments, oil, natural organic matter and color are separated from process liquids. A clear process liquid can be provided by a clarifier. A purifier is a centrifugal separator for liquid - liquid - solid separation, in which two liquids of different densities and solids can be separated from each other. For example, water, oil, and fines can be separated from each other. Using a purifier, the light liquid phase is typically the large fraction which is meant to cleaned. A concentrator is a centrifugal separator designed to separate three different phases, one solid phase and two liquid phases of different densities and clean the densest/heaviest liquid phase.

In the method according to the present disclosure, the plant-based raw material is edible, protein-containing material. The raw material may be for example legumes, oilseeds or cereals. Some of the legumes or oilseeds may have a fat content of more than 10% by weight, which is herein referred to as a high fat content. Before providing a protein suspension, at least part of the fats may be removed from the raw material. Examples of raw materials having a high fat content include oilseeds of soybean, sunflower, rapeseed, cottonseed, lupin and linseed, but are not limited thereto. Combinations of these raw materials may also be used. The raw materials may alternatively be legumes or cereals, which may have a fat content of 10% by weight or less, which is herein referred to as a low fat content. Examples of raw materials having a low fat content are yellow peas, fava beans, mung beans, lentils and chickpeas, but the raw-materials are not limited thereto. Combinations of these raw materials may also be used.

A plant-based protein suspension comprising suspended protein particles, which is concentrated according to the method of the present disclosure, may be provided in different ways. Firstly, the plant-based raw material may be provided in the form of a protein meal or flour. The fat-content of the protein meal or flour may be reduced. The meal or flour may be provided by any suitable method known in the art. The meal can be mixed with water and the plant-based protein may be dissolved and after removal of non-soluble/non-protein components, the protein is precipitated to a fine particulate suspension.

By suspension is meant is a heterogeneous mixture of a finely distributed solid in a liquid. The solid is not dissolved in the liquid.

According to an example, the plant-based protein can be dissolved by adding an alkali to a ground plant-based meal, which may be provided as an aqueous mixture. The alkali may be for example an alkali metal hydroxide, such as sodium hydroxide (lye). The plant-based protein can be precipitated by adding an acid or an organic solvent to the solution comprising the dissolved plant-based protein. The acid may be an inorganic acid, such as hydrochloric acid or an organic acid or an organic solvent, such as ethanol. The acid may be a weak acid. The acid can be added at a pH corresponding to the isoelectric point of the alkali-soluble proteins in the meal. Other known methods to provide the plant-based protein suspension may be used, if the level of precipitated dissolved proteins is sufficient.

According to the present method, use is made of a high-speed centrifugal separator, which comprises a frame, a drive member and a centrifuge bowl to concentrate the protein suspension. The drive member is configured to rotate the centrifuge bowl in relation to the frame around an axis of rotation. The centrifuge bowl encloses a separation space comprising a stack of separation discs, an inlet for receiving the plant-based protein suspension, a liquid light phase outlet for a separated liquid light phase and a heavy phase outlet for the separated concentrated protein suspension as a heavy phase. The high-speed centrifugal separator may comprise at least one sludge or solids outlet for discharging a separated solid phase. The solid outlet is arranged at the periphery of the centrifuge bowl. The discharge may be done intermittently.

The high-speed centrifugal separator used in the present method comprises a flow influencing or regulating means in fluid connection with either one or both of the heavy phase outlet and light phase outlet. By flow influencing means is meant a device that is configured to regulate the flow characteristics of the separated fluids at the outlets. For example, an active type of device may be used to regulate the counter pressure of the outlet, an example of which is a valve. Passive-type of devices may be used as well, for example self-regulating vortex nozzles, which affect the linearity of the flow at the outlets.

According to the present method the plant-based protein suspension is fed to the inlet of the high-speed centrifugal separator and is separated into a liquid light phase, which comprises a clarified liquid phase of the suspension, and a heavy phase comprising the concentrated protein suspension with the protein particles. In the method the removal of the concentrated plant-based protein suspension as the heavy phase through the heavy phase outlet is possible, since the removal or discharge through the respective outlet is affected by means of the liquid flow influencing means so that the suspended particles can be pushed through the heavy phase outlet. At the same time the liquid light phase removed through the light phase outlet preferably contains no or only a low amount of suspended protein particles, such as less than 2% by volume.

The high-speed separator usable in the method according to the present disclosure will be further illustrated by the following description with reference to the accompanying drawings.

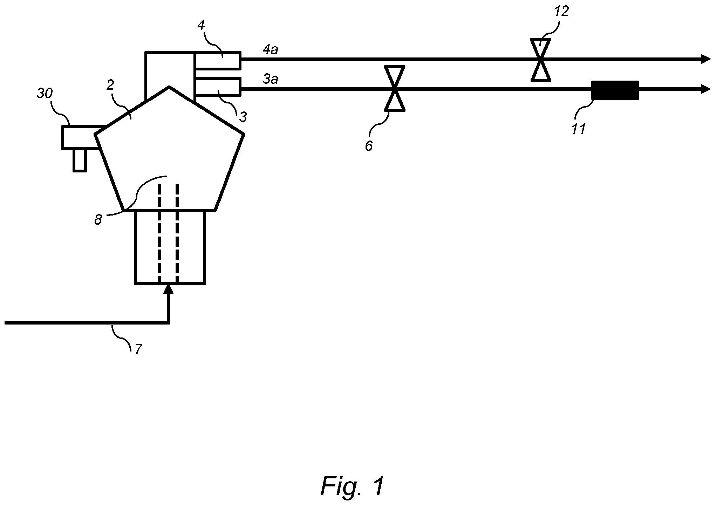

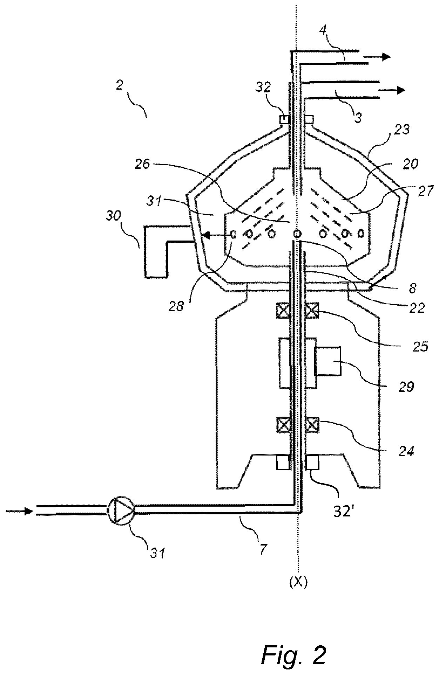

Fig. 1 shows schematically a high-speed centrifugal separator of the type that can be used in the present method for concentrating a plant-based protein suspension. The method may be continuous or a batch method. The plant-based protein suspension is fed by a pump via an inlet pipe 7 to a high-speed centrifugal separator 2, which may be mechanically hermetically sealed. The feed is introduced centrally from below, i.e., the separator is bottom-fed, and enters the separation space in the separator via an inlet 8. A more detailed description of the working principles of the separator 2 is disclosed in relation to Fig. 2 below.

After being processed in the separator 2, the suspension comprising particulate plant-based proteins is separated into a heavy phase comprising the concentrated suspended plant-based proteins and to a liquid light phase. Since the heavy phase comprises the particles, it may have a higher density than the liquid light phase. The particles are suspended in the liquid forming the light phase. The heavy phase is removed from the separator via a heavy phase outlet 3. A clarified light phase is removed via a liquid light phase outlet 4. The heavy phase outlet 3 comprises an outlet pipe 3a, which fluidly connects the outlet 3 to a regulating valve 6, which is configured to influence or regulate the flow through the outlet 3 by regulating the counter pressure of the outlet 3, suitably with respect to the liquid light phase outlet 4. In the similar manner, the liquid light phase outlet 4 comprises an outlet pipe 4a, which fluidly connects the outlet 4 to a regulating valve 12. The regulating valve corresponds to the flow influencing means and is configured to regulate the counter pressure of the light phase outlet 4, suitably with respect to the liquid heavy phase outlet 3.

The regulation of the valves can be determined by a laboratory spin-test of the light phase If the volumetric losses of the protein particles exceed predefined volumetric losses, e.g. more than 1%, the counter pressure at the heavy phase and/or the light phase is adjusted by means of the valves. The sampling may be done manually, for example by taking a sample is taken manually, spinning the sample, and then evaluating the content of suspended particles by volume %. The counter pressure is then regulated based on the content of suspended particles in the light phase. The content should be as low as possible to minimize product losses and securing highest possible concentration of heavy phase. The sampling can be also performed automatically by using a suitable sensor connected with any of the outlets 3 and/or 4. The measured characteristic of the liquid phase can be in-line and the measurement value can be sent to a control unit, which compares the measured value with a reference value and depending on the comparison, adjusts the counter pressure of one of the outlets with respect to the other outlet by the regulating valves 6, 12.

By regulating the valve 6, the counter pressure of outlet 3 is adjusted by varying the opening degree of the valve. In this way the interface level between the separated heavy phase and the light phase in the separator is adjusted radially. Thus, the opening degree of the valve 6 determines the counter pressure and by regulating valve 6, the content of suspended proteins present in the separated heavy phase may be varied or controlled. By regulating the valves 6 and 12 so that the counter pressure at the heavy phase and the light phase outlets is adjusted, the volumetric split between the light and heavy phase can be adjusted By adjusting the counter pressure at each of the heavy phase and light phase outlets, the volumetric split is adjusted such that the loss of the suspended protein to the light phase is limited. The objective is at the same time to maximize the concentration of the suspended protein in the heavy phase, while it still can be pressed out from the heavy phase outlet without blocking the outlet.

Furthermore, a flow transmitter 11 can be arranged downstream on pipe 3a as compared to the regulating valve 6. The flow transmitter 11 measures the flow through the outlet pipe 3a. The flow transmitter may be used to contribute to the detection of a situation where the separator bowl is clogged with the suspension. The flow transmitter may be used to automatically control that the flow through the outlet pipe 3a is changed upon a change in the control valve 6.

Suitable separator type for the use in the present invention is disclosed by GB1111557. Another example of a centrifugal separator suitable for the method according to the invention is depicted in Fig. 2.

The centrifugal separator 2 comprises a rotor 20 (bowl) arranged for rotation about an axis of rotation X by means of a spindle 22. The spindle 22 is supported in the frame 23 of the centrifugal separator in a bottom bearing 24 and a top bearing 25. The bowl 20 forms within itself a separation chamber 26 in which centrifugal separation of the suspended plant-based protein takes place during operation. The centrifugal separator may be of a hermetically sealed type with a closed separation space 26, i.e., the separation space 26 is intended to be filled with liquid during operation. This means that no air or free liquid surfaces is meant to be present in the bowl. The separator may comprise hermetic mechanical seals at the inlet and/or the outlets. The separation space 26 is provided with a stack of -conical separation discs 27 to achieve effective separation of the fluid. The stack of truncated conical separation discs 27 are examples of surface-enlarging inserts. These discs 27 are fitted centrally and coaxially with the rotor and comprise holes which form channels for axial flow of liquid when the separation discs 27 are fitted in the centrifugal separator.

An inlet 8 for introducing the suspension comprising plant-based proteins for centrifugal separation extends into the bowl, providing the material to be separated to the separation space 26. The inlet 8 extends through the spindle 22, which takes the form of a hollow, tubular member. Introducing the liquid material from the bottom provides a gentle acceleration of the liquid. The inlet 8 is further connected to an inlet pipe 7, into which pipe the suspension comprising plant-based proteins to be separated is pumped by means of pump 31.

The rotor has extending from it a liquid light phase outlet 4 for a lower density component separated from the suspension. A heavy phase outlet 3 for the concentrated protein suspension is arranged radially outwards of the light phase outlet. The outlets 3 and 4 extend through the casing 23. The space 31 is sealed by a seal 32 on the top and a seal 32' at the bottom. The rotor is provided at its outer periphery with a set of radial solid phase outlets 28 in the form of intermittently openable outlets for discharge of e.g., solids and/or a higher density component in the suspension comprising plant-based proteins. This material is thus discharged from a radially outer portion of the separation chamber 26 to the space 31 round the rotor.

The centrifugal separator 2 is further provided with a drive motor 29. This motor 29 may for example comprise a stationary element and a rotatable element, which rotatable element surrounds and is so connected to the spindle 22 that during operation it transmits driving torque to the spindle 22 and hence to the bowl 20. The drive motor may be an electric motor. Furthermore, the drive motor 29 may be connected to the spindle 22 by transmission means. The transmission means may be in the form of a worm gear which comprises a pinion and an element connected to the spindle to receive driving torque. The transmission means may alternatively take the form of a propeller shaft, drive belts or the like, and the drive motor may alternatively be connected directly to the spindle.

The centrifugal separator may further comprise a vessel 30 in the form of a cyclone connected to the space 31 and adapted to gather solids and liquid from the solid outlets 28. The gathering vessel 30 is further connected to a discharge device in the form of a sludge pump for discharge of solids and liquid present in the gathering vessel. The sludge pump is provided with a check valve function which prevents flow back into the vessel via the sludge pump.

During operation of the separator in Fig. 2, the rotor 20 is caused to rotate by torque transmitted from the drive motor 29 to the spindle 22. Via the inlet 8, the suspension comprising plant-based protein particles is brought into the separation space 26. In the hermetic type of inlet, the acceleration of the liquid suspension is initiated at a small radius and is gradually increased while the liquid leaves the inlet and enters the separation space 26. Different phases in the suspension, i.e., a clarified liquid corresponding to the light phase and heavy phase containing the suspended particles, are separated between the separation discs 27 fitted in the separation space 26. Heavier components in the suspension, i.e., the suspended proteins, move radially outwards between the separation discs, whereas the clarified liquid of the suspension, i.e., the light phase, moves radially inwards between the separation discs and is forced through outlet 4 that is arranged at the radial innermost level in the separator. The concentrated suspension may have higher density and is instead forced out through the outlet 3 that is at a radial level that is larger than the radial level of outlet 4. Thus, during separation, an interphase between the concentrated suspension and the clarified liquid of the suspension is formed in the separation space 26. The radial level, i.e., the distance from rotation al axis X, of this interface level can be in the hermetic separator determined by the counter pressure of outlets 3 and 4 of the separator. Some of the solids also accumulate within the solids phase outlets 28. The solids are emptied intermittently from the separation space by the solid outlets 28 being opened, whereupon solids and a certain amount of fluid is discharged from the separation space by means of centrifugal force. Solids which are discharged from the separation space via the solid outlets is conveyed from the surrounding space 31 to the gathering vessel 30 connected thereto, in which the solids accumulate and from which it is pumped out by a sludge pump.

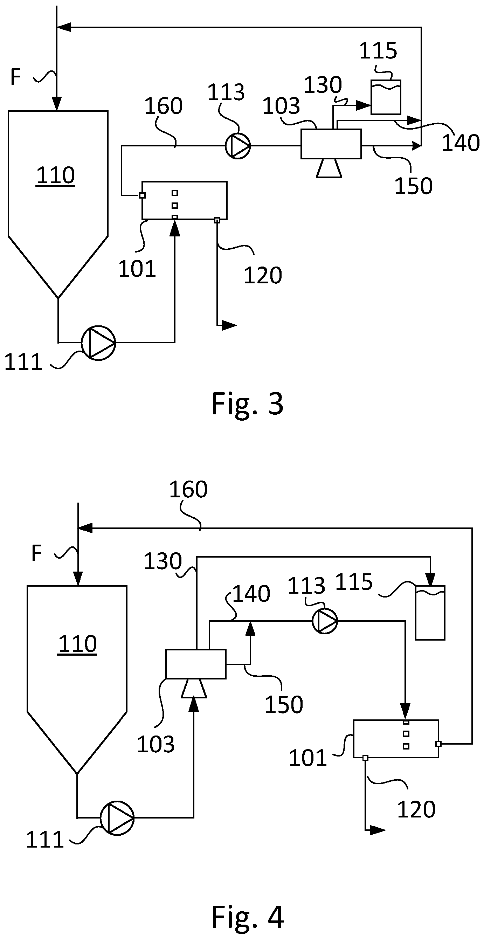

Fig. 3 and 4 illustrate example modes of the method, wherein an unconcentrated protein suspension F is fed to a tank 110. The suspension F is then fed from the tank 110 by means of a pump 111 to a first separation step.

In Fig. 3, the first separation step is performed in a decanter separator 101. The suspension F is thus first pumped to a separation step in the decanter separator 101, which is arranged upstream of a high-speed separator 103. In the decanter 101 the protein suspension is separated by centrifugal force so that part of the suspended proteins 120 is harvested at a solids outlet of the decanter and a liquid phase 160 still containing suspended particles is pumped from the decanter 101 by means of a pump 113 to the high-speed separator 103. A clarified light phase 130 is collected in a tank 115. The heavy phase 140 together with any solid matter 150 discharged from the bowl periphery of the high-speed separator 103, is fed back to the decanter 101, either directly or via the feed tank 110 as illustrated in Fig. 3

In Fig. 4 the first separation step is performed in a high-speed separator and the protein suspension F is thus first fed to the high-speed separator 103. The clear light phase 130 is collected into a tank 115. The heavy phase 140 comprising the suspended proteins and discharged proteins in the solids 150 are pumped by means of a pump 113 to a decanter 101. After centrifugation in the decanter 101, the suspended proteins 120 are collected and a liquid phase 160 still containing suspended particles is returned to the tank.

### Examples

### Pre-treatment to obtain suspension

A slurry is prepared by mixing dehulled flour of yellow pea with water. The pH of the resulting slurry is modified by adding lye to a pH value higher than 7, which accelerates the protein solubilization. After the pH adjustment, 30-60 minutes of residence time is provided to provide time for extracting the water-soluble proteins.

Once the extraction is completed, the resulting slurry will be pumped to a centrifugal separation unit such as a decanter, hydrocyclones etc, where the suspended particles comprising starch and fibres are removed from the liquid that includes the solubilized proteins.

The clarified liquid from the previous step is collected into an agitated tank, where the pH is lowered to the isoelectric point of the proteins with the dosing of an acid. The decrease in pH lowers the solubility of the proteins and causes them to precipitate out of the solution. Thereby a suspension comprising plant-based proteins from yellow peas is provided.

### Suspension characteristics

### Particle size distribution

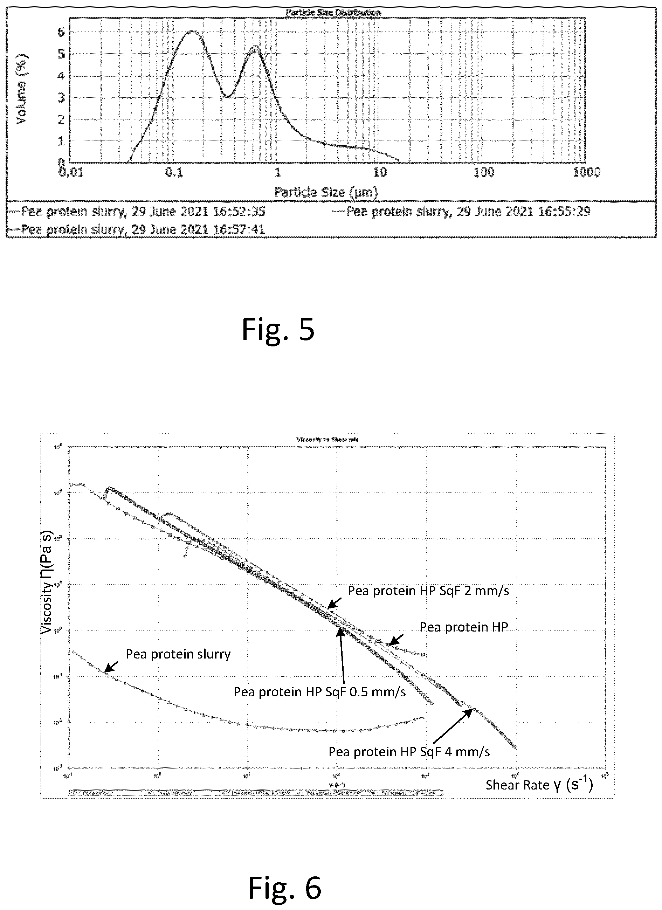

Particle size distribution of the suspended yellow pea protein was measured by a Malvern Mastersizer 2000, which is a laser diffraction instrument. Three measurements were made and Fig. 5 shows the results. It can be seen that the suspension comprises fine particles, where *d(0,1): 0,089 µm, d(0,5): 0,286 µm and d(0,9):* 1,779 *µm.*

### Shear Viscosity

The shear viscosity was measured by a rheometer for both the slurry (suspension) and for solids collected from centrifugation for 10 min at 3000 rpm. The measurements were performed by Malvern Kinexus Lab+ Rheometer and the shear viscosity measurement sequency are from the rheometer rSpace software. The purpose of the measurement was to see if the slurry and solids have shear thinning properties.

Slurry was measured using bob & cup geometries with the shear rate ramp sequence. The solids were measured using the serrated plate geometries with the shear rate ramp sequence. For further flow characterization of the solids also squeeze flow sequence was applied with parallel plates and gapping speeds 0,5; 2 and 4 mm/s (Pea protein HP; Pea protein HP SqF 0.5; 2; 4 mm/s).

Shear viscosity of both slurry (Pea protein slurry) and solids are presented in Fig. 6. It can be seen from the Fig. 6 that both slurry and solids show shear thinning behaviour.

### Comparative tests

Comparative tests were performed with the plant-based protein suspension prepared as described above.

In the tests, a volumetric solids content of 0.5% was a target value for the light phase, i.e. the clarified liquid phase of the suspension from both the high-speed separator and the decanter separator. A feed volume comprising 120 I/h solids could be processed in a decanter, while a feed flow comprising 700 I/h solids could be treated in Alfa Laval PurePulp^{®} high-speed separator. This means that the volumetric capacity of the high-speed separator was 5.8 times larger than that of a decanter, while the same low content of suspended particles in the light phase was obtained (0,5% vol) and no clogging of the machines occurred.

### Conclusions from the plant-based protein separation tests

Pilot scale test on yellow pea has shown, that Alfa Laval PurePulp^{®} high-speed separator can harvest protein precipitate and deliver a light phase with a very low content of suspended particles.

The high-speed separator is thus a viable technology for separating suspended plant-based protein as the suspended particles due to their physicochemical nature can be pushed to the heavy phase outlet. This enables to run the high-speed separator (HSS) with a continuous heavy liquid flow, thus allowing to feed the machine with feed concentrations that are normally considered too high for HSS. The machine has still solids discharge capability, which is used to maintain the stability of the machine since there will be accumulation of impurities in the periphery. The discharge is not the intended separation method as opposed to other types of HSS machines such as clarifiers.

The Alfa Laval PurePulp^{®} high-speed separator demonstrates higher volumetric capacity with similar or improved light phase quality than the decanter centrifuge. This allows to use the Alfa Laval PurePulp^{®} high-speed separator either as a pre-concentrator prior to the decanter or as a light phase cleaner after the decanter. With this combination it is possible to use less decanters and have overall higher recovery of the protein within the overall process.

## Claims

1. A method of recovering a concentrated plant-based protein suspension, comprising:
providing a plant-based protein suspension comprising suspended particles,
- by dissolving a plant-based protein and by precipitating the dissolved protein to provide the plant-based protein suspension,
- providing a high-speed centrifugal separator (2) which comprises:
a frame (23), a drive member (29) and a centrifuge bowl (20),
wherein the drive member (29) is configured to rotate the centrifuge bowl in relation to the frame (23) around an axis of rotation (X), and
wherein the centrifuge bowl (20) encloses a separation space (26) comprising a stack of separation discs (27), and
wherein the centrifuge bowl (20) further comprises an inlet (8) for receiving the plant-based protein suspension, a liquid light phase outlet (4) for a separated liquid light phase and a heavy phase outlet (3) for a separated heavy phase, and
wherein the heavy phase outlet and/or light phase outlet is arranged in fluid connection with a flow influencing means (6; 12), and
wherein the method further comprises:
- feeding the plant-based protein suspension to the inlet of the high-speed centrifugal separator,
- separating the plant-based protein suspension into a liquid light phase and a heavy phase, which comprises the concentrated plant-based protein suspension,
- removing the concentrated plant-based protein suspension as the heavy phase flow through the heavy phase outlet by influencing the flow by means of the flow influencing means.

2. Method according to claim 1, wherein the step of influencing the flow through the heavy phase outlet comprises adjusting counter pressure of the heavy phase at the heavy phase outlet with respect to the liquid light phase outlet, or vice versa, and wherein the flow influencing means is a flow regulating means comprising a valve.

3. Method according to claim 1, wherein the influencing of the flow through the heavy phase outlet comprises passively regulating the flow by means of a passive flow regulator at the heavy phase outlet.

4. Method according to any one of the preceding claims, wherein the method further comprises
- measuring at least one parameter of the removed heavy phase and/or liquid light phase, wherein said parameter is related to the concentration of the heavy phase in the light phase, or vice versa; and
- adjusting the counter pressure of the heavy phase outlet with respect to the liquid light phase outlet, or vice versa, based on the parameter related to the concentration.

5. Method according to any one of the previous claims comprising concentrating the plant-based protein suspension in at least two sequential centrifugal separation steps, wherein one of the two centrifugal separation steps is performed in a decanter centrifuge.

6. Method according to claim 5, wherein a first step of the centrifugal separation is performed in the high-speed centrifugal separator and a second step is performed in the decanter centrifuge.

7. Method according to claim 5, wherein a first step of the centrifugal separation is performed in the decanter centrifuge and a second step is performed in the high-speed centrifugal separator.

8. Method according to any one of claims 1-7, wherein plant-based protein is dissolved by adding an alkali to a ground plant-based meal.

9. Method according to claim 8, wherein the plant-based protein is precipitated by adding an acid or an organic solvent, such as ethanol, to the dissolved plant-based protein.

10. Method according to any one of the preceding claims, wherein no salt is added to the plant-based protein suspension after precipitation and before separation into a liquid light phase and a liquid heavy phase.

11. Method according to any one of the preceding claims, wherein the high-speed centrifugal separator in the step of separating is hermetically sealed.

12. Method according to any one of the preceding claims, wherein the plant-based raw material for the suspension comprising plant-based proteins comprises low-fat or high-fat plant-based raw materials, such as legumes, oilseeds or cereals.

13. Method according to claim 12, wherein the plant-based raw material comprises low-fat raw materials comprising legumes, such as yellow peas, fava beans, mung beans, lentils, chickpeas, or combinations thereof.

14. Method according to claim 12, wherein the plant-based raw material comprises high-fat raw materials comprising-oilseeds, such as oilseeds of soybean, lupin, sunflower, rapeseed, cottonseed, linseed or combinations thereof.

15. Method according to any one of the preceding claims, comprising de-oiling the plant-based raw material before providing the plant-based protein suspension comprising the suspended protein particle

## Patentansprüche

1. Verfahren zum Rückgewinnen einer konzentrierten Proteinsuspension auf Pflanzenbasis, das Folgendes umfasst:
Bereitstellen einer Proteinsuspension auf Pflanzenbasis, die suspendierte Teilchen umfasst,
- durch Auflösen eines Proteins auf Pflanzenbasis und durch Ausfällen des aufgelösten Proteins, um die Proteinsuspension auf Pflanzenbasis bereitzustellen,
- Bereitstellen eines Hochgeschwindigkeitszentrifugalabscheiders (2), der Folgendes umfasst:
einen Rahmen (23), ein Antriebselement (29) und eine Zentrifugentrommel (20),
wobei das Antriebselement (29) dafür konfiguriert ist, die Zentrifugentrommel im Verhältnis zu dem Rahmen (23) um eine Drehachse (X) zu drehen, und
wobei die Zentrifugentrommel (20) einen Abscheidungsraum (26) umschließt, der einen Stapel von Scheidetellern (27) umfasst, und
wobei die Zentrifugentrommel (20) ferner einen Einlass (8) zum Aufnehmen der Proteinsuspension auf Pflanzenbasis, einen Flüssigleichtphasenauslass (4) für eine abgeschiedene flüssige leichte Phase und einen Schwerphasenauslass (3) für eine abgeschiedene schwere Phase umfasst und
wobei der Schwerphasenauslass und/oder der Flüssigleichtphasenauslass in Fluidverbindung mit einem Durchflussbeeinflussungsmittel (6; 12) angeordnet sind und
wobei das Verfahren ferner Folgendes umfasst:
- Einspeisen der Proteinsuspension auf Pflanzenbasis in den Einlass des Hochgeschwindigkeitszentrifugalabscheiders,
- Trennen der Proteinsuspension auf Pflanzenbasis in eine flüssige leichte Phase und eine schwere Phase, welche die konzentrierte Proteinsuspension auf Pflanzenbasis umfasst,
- Entnehmen der konzentrierten Proteinsuspension auf Pflanzenbasis als den Schwerphasenstrom durch den Schwerphasenauslass durch Beeinflussen des Durchflusses mit Hilfe des Durchflussbeeinflussungsmittels.

2. Verfahren nach Anspruch 1, wobei der Schritt des Beeinflussens des Durchflusses durch den Schwerphasenauslass das Anpassen eines Gegendrucks der schweren Phase an dem Schwerphasenauslass in Bezug auf den Flüssigleichtphasenauslass oder umgekehrt umfasst und wobei das Durchflussbeeinflussungsmittel ein Durchflussregelungsmittel ist, das ein Ventil umfasst.

3. Verfahren nach Anspruch 1, wobei das Beeinflussen des Durchflusses durch den Schwerphasenauslass das passive Regulieren des Durchflusses mit Hilfe eines passiven Durchflussreglers an dem Schwerphasenauslass umfasst.

4. Verfahren nach einem der vorhergehenden Ansprüche, wobei das Verfahren ferner Folgendes umfasst:
- Messen mindestens eines Parameters der entnommenen schweren Phase und/oder der flüssigen leichten Phase, wobei der Parameter mit der Konzentration der schweren Phase in der leichten Phase oder umgekehrt verknüpft ist,
- Anpassen des Gegendrucks des Schwerphasenauslasses in Bezug auf den Flüssigleichtphasenauslass oder umgekehrt, auf Grundlage des Parameters, der mit der Konzentration verknüpft ist.

5. Verfahren nach einem der vorhergehenden Ansprüche, welches das Konzentrieren der Proteinsuspension auf Pflanzenbasis in mindestens zwei aufeinander folgenden Zentrifugalabscheidungsschritten umfasst, wobei einer von den zwei Zentrifugalabscheidungsschritten in einer Dekanterzentrifuge durchgeführt wird.

6. Verfahren nach Anspruch 5, wobei ein erster Schritt der Zentrifugalabscheidung in dem Hochgeschwindigkeitszentrifugalabscheider durchgeführt wird und ein zweiter Schritt in der Dekanterzentrifuge durchgeführt wird.

7. Verfahren nach Anspruch 5, wobei ein erster Schritt der Zentrifugalabscheidung in der Dekanterzentrifuge durchgeführt wird und ein zweiter Schritt in dem Hochgeschwindigkeitszentrifugalabscheider durchgeführt wird.

8. Verfahren nach einem der Ansprüche 1 bis 7, wobei das Protein auf Pflanzenbasis durch Hinzugeben eines Alkalis zu einem gemahlenen Mehl auf Pflanzenbasis aufgelöst wird.

9. Verfahren nach Anspruch 8, wobei das Protein auf Pflanzenbasis durch Hinzugeben einer Säure oder eines organischen Lösungsmittels, wie beispielsweise Ethanol, zu dem aufgelösten Protein auf Pflanzenbasis ausgefällt wird.

10. Verfahren nach einem der vorhergehenden Ansprüche, wobei zu der Proteinsuspension auf Pflanzenbasis nach der Ausfällung und vor der Trennung in eine flüssige leichte Phase und eine flüssige schwere Phase kein Salz hinzugefügt wird.

11. Verfahren nach einem der vorhergehenden Ansprüche, wobei der Hochgeschwindigkeitszentrifugalabscheider in dem Schritt des Abscheidens hermetisch abgedichtet ist.

12. Verfahren nach einem der vorhergehenden Ansprüche, wobei das Rohmaterial auf Pflanzenbasis für die Suspension, die Protein auf Pflanzenbasis umfasst, fettarme oder fettreiche Rohmaterialien auf Pflanzenbasis, wie beispielsweise Hülsenfrüchte, Ölsaaten oder Getreide, umfasst.

13. Verfahren nach Anspruch 12, wobei das Rohmaterial auf Pflanzenbasis fettarme Rohmaterialien umfasst, die Hülsenfrüchte, wie beispielsweise gelbe Erbsen, Ackerbohnen, Mungbohnen, Linsen, Kichererbsen oder Kombinationen derselben, umfassen.

14. Verfahren nach Anspruch 12, wobei das Rohmaterial auf Pflanzenbasis fettreiche Rohmaterialien umfasst, die Ölsaaten, wie beispielsweise Ölsaaten von Sojabohne, Lupine, Sonnenblume, Raps, Baumwollsamen, Leinsamen oder Kombinationen derselben, umfassen.

15. Verfahren nach einem der vorhergehenden Ansprüche, das vor dem Bereitstellen der Proteinsuspension auf Pflanzenbasis, die das suspendierte Proteinteilchen umfasst, das Entölen des Rohmaterials auf Pflanzenbasis umfasst.

## Revendications

1. Procédé de récupération d'une suspension de protéine à base de plante concentrée, comprenant :
la fourniture d'une suspension de protéine à base de plante comprenant des particules en suspension,
- en dissolvant une protéine à base de plante et en précipitant la protéine dissoute pour fournir la suspension de protéine à base de plante,
- en fournissant un séparateur centrifuge à grande vitesse (2) qui comprend :
une armature (23), un élément d'entraînement (29) et un bol centrifuge (20),
dans lequel l'élément d'entraînement (29) est configuré pour faire tourner le bol centrifuge par rapport à l'armature (23) autour d'un axe de rotation (X), et
dans lequel le bol centrifuge (20) renferme un espace de séparation (26) comprenant une pile de disques de séparation (27), et
dans lequel le bol centrifuge (20) comprend en outre une entrée (8) destinée à recevoir la suspension de protéine à base de plante, une sortie de phase légère liquide (4) pour une phase légère liquide séparée et une sortie de phase lourde (3) pour une phase lourde séparée, et
dans lequel la sortie de phase lourde et/ou la sortie de phase légère sont agencées en connexion fluidique avec un moyen d'influence de l'écoulement (6 ; 12), et
dans lequel le procédé comprend en outre :
- l'alimentation de l'entrée du séparateur centrifuge à grande vitesse avec la suspension de protéine à base de plante,
- la séparation de la suspension de protéine à base de plante en une phase légère liquide et une phase lourde, qui comprend la suspension de protéine à base de plante concentrée,
- l'enlèvement de la suspension de protéine à base de plante concentrée en tant qu'écoulement de phase lourde à travers la sortie de phase lourde en influençant l'écoulement au moyen du moyen d'influence de l'écoulement.

2. Procédé selon la revendication 1, dans lequel l'étape d'influence de l'écoulement à travers la sortie de phase lourde comprend l'ajustement d'une contre-pression de la phase lourde au niveau de la sortie de phase lourde par rapport à la sortie de phase légère liquide, ou inversement, et dans lequel le moyen d'influence d'écoulement est un moyen de régulation d'écoulement comprenant une vanne.

3. Procédé selon la revendication 1, dans lequel l'influence de l'écoulement à travers la sortie de phase lourde comprend la régulation passive de l'écoulement au moyen d'un régulateur d'écoulement passif au niveau de la sortie de phase lourde.

4. Procédé selon l'une quelconque des revendications précédentes, dans lequel le procédé comprend en outre
- la mesure d'au moins un paramètre de la phase lourde enlevée et/ou de la phase légère liquide, dans lequel ledit paramètre est lié à la concentration de la phase lourde dans la phase légère, ou inversement ; et
- l'ajustement de la contre-pression de la sortie de phase lourde par rapport à la sortie de phase légère liquide, ou inversement, sur la base du paramètre lié à la concentration.

5. Procédé selon l'une quelconque des revendications précédentes comprenant la concentration de la suspension de protéine à base de plante dans au moins deux étapes de séparation centrifuge séquentielles, dans lequel l'une des deux étapes de séparation centrifuge est réalisée dans un centrifugeur décanteur.

6. Procédé selon la revendication 5, dans lequel une première étape de la séparation centrifuge est réalisée dans le séparateur centrifuge à grande vitesse et une deuxième étape est réalisée dans le centrifugeur décanteur.

7. Procédé selon la revendication 5, dans lequel une première étape de la séparation centrifuge est réalisée dans le centrifugeur décanteur et une deuxième étape est réalisée dans le séparateur centrifuge à grande vitesse.

8. Procédé selon l'une quelconque des revendications 1 à 7, dans lequel la protéine à base de plante est dissoute en ajoutant un alcali à un repas végétal mouliné.

9. Procédé selon la revendication 8, dans lequel la protéine à base de plante est précipitée en ajoutant un acide ou un solvant organique, tel que l'éthanol, à la protéine à base de plante dissoute.

10. Procédé selon l'une quelconque des revendications précédentes, dans lequel aucun sel n'est ajouté à la suspension de protéine à base de plante après la précipitation et avant la séparation en une phase légère liquide et une phase lourde liquide.

11. Procédé selon l'une quelconque des revendications précédentes, dans lequel le séparateur centrifuge à grande vitesse lors de l'étape de séparation est scellé hermétiquement.

12. Procédé selon l'une quelconque des revendications précédentes, dans lequel la matière première à base de plante pour la suspension comprenant des protéines à base de plante comprend des matières premières à faible teneur en matières grasses ou à teneur élevée en matières grasses, telles que des légumineuses, des graines oléagineuses ou des céréales.

13. Procédé selon la revendication 12, dans lequel la matière première à base de plante comprend des matières premières à faible teneur en matières grasses comprenant des légumineuses, telles que les pois jaunes, les fèves, les haricots mungo, les lentilles, les pois chiches ou des combinaisons de ceux-ci.

14. Procédé selon la revendication 12, dans lequel la matière première à base de plante comprend des matières premières à forte teneur en matière grasse comprenant des graines oléagineuses, telles que des graines oléagineuses de soja, de lupin, de tournesol, de colza, de graines de coton, de graines de lin ou des combinaisons de celles-ci.

15. Procédé selon l'une quelconque des revendications précédentes, comprenant l'extraction de l'huile de la matière première à base de plante avant la fourniture de la suspension de protéine à base de plante comprenant la particule de protéine en suspension.
